Europäisches Patentamt

European Patent Office

Office européen des brevets

(19)

(11) Veröffentlichungsnummer : **0 069 252**
**B1**

(12) **EUROPÄISCHE PATENTSCHRIFT**

(45) Veröffentlichungstag der Patentschrift :
22.10.86

(21) Anmeldenummer : 82105224.8

(22) Anmeldetag : 15.06.82

(51) Int. Cl.⁴ : **A 61 F   2/32**

(54) **Gelenkendoprothese.**

(30) Priorität : 30.06.81 DE 3125657
03.05.82 DE 3216533

(43) Veröffentlichungstag der Anmeldung :
12.01.83 Patentblatt 83/02

(45) Bekanntmachung des Hinweises auf die Patenterteilung : 22.10.86 Patentblatt 86/43

(84) Benannte Vertragsstaaten :
**AT BE CH DE FR GB IT LI NL SE**

(56) Entgegenhaltungen :
**EP-A- 0 012 146**
**DE-A- 2 839 093**
**DE-A- 2 931 750**
**FR-A- 1 046 516**
**FR-A- 1 083 013**
**FR-A- 1 416 534**
**US-A- 3 228 393**
**US-A- 3 740 769**
**US-A- 4 068 324**

(73) Patentinhaber : **Waldemar Link GmbH & Co**
**Barkhausenweg 10**
**D-2000 Hamburg 63 (DE)**

(72) Erfinder : **Link, Helmut D.**
**Wildstieg 14**
**D-2000 Hamburg 65 (DE)**
Erfinder : **Keller, Arnold**
**An der Naherfurth 5**
**D-2061 Kayhude (DE)**

(74) Vertreter : **Glawe, Delfs, Moll & Partner Patentanwälte**
**Postfach 26 01 62 Liebherrstrasse 20**
**D-8000 München 26 (DE)**

**Beschreibung**

Die Erfindung bezieht sich auf eine Gelenkendoprothese mit einem in die Höhle eines langen Knochens einzusetzenden Schaft und einer den Schaft an dessen dem Gelenk benachbarten Ende abschließenden Auflageplatte, deren Unterfläche zur Auflage auf der formentsprechend vorbearbeiteten Resektionsfläche des Knochens bestimmt ist und in ihrem medialen Teil einen durchschnittlich größeren Winkel mit der Richtung des langen Knochens einschließt als in ihrem lateralen Teil.

Bei den meisten bekannten Hüftgelenkendoprothesen ist die Unterfläche der Auflageplatte eben. Da sie schräg zur Hauptbelastungsrichtung liegt, ist ihre Fähigkeit zur Kraftübertragung begrenzt. Überdies neigt sie aufgrund des Effekts der schiefen Ebene dazu, eine medial gerichtete Relativbewegung der Prothese gegenüber dem Knochen hervorzurufen. Um dies zu verhindern, hat man die Unterfläche der Auflageplatte auch schon mit Riefen oder Zähnen versehen, die beim Einsetzen der Prothese in die Knochenoberfläche eingepreßt werden und damit eine Verankerung gegen seitliche Relativbewegungen ergeben sollen (DE-A-28 39 093). In der Praxis läßt sich dieses Ziel allerdings nur begrenzt erreichen, weil es sehr schwer ist, die Prothese bei der Operation so stark gegen die Resektionsfläche zu pressen, daß die Zähne hinreichend in die harte Corticalis eindringen. Es ist auch schwierig, die Resektionsfläche so genau zu formen, daß bei vorschriftsmäßigem Sitz der Prothese die Unterfläche der Auflageplatte genau parallel zur Resektionsfläche liegt, so daß sämtliche Zähne am Eingriff gleichmäßig beteiligt werden können.

Es ist eine Hüftgelenkendoprothese bekannt (DE-A-29 31 750), bei welcher die durchgehend eben geformte Unterfläche der Auflageplatte weniger stark geneigt verlaufen soll, als dies sonst üblich ist. Jedoch erscheint die Realisierbarkeit dieses Vorschlags höchst fraglich, weil die Neigungsrichtung der Resektionsfläche nicht frei wählbar, sondern vorgegeben ist durch die Lage des großen und kleinen Trochanter, an denen sie proximal benachbart entlanggeführt werden muß, so daß sich bei durchgehend ebener Auflageplatte im allgemeinen ein Neigungswinkel von etwa 45° ergibt.

Ein geringerer Neigungswinkel läßt sich nur dann erreichen (FR-A-1 416 534), wenn die mit der Resektionsfläche des Knochens zusammenwirkende Unterfläche der Auflageplatte winklig aus Teilflächen zusammengesetzt wird, von denen die mediale etwa horizontal verläuft, während die laterale, dem großen Trochanter zugewendete Teilfläche steil ansteigt. Jedoch hat diese Formgebung den Nachteil, daß zum einen eine wesentlich größere Knochenmenge reseziert werden muß als bei der üblichen Resektion schräg durchgehend etwa vom großen zum kleinen Trochanter. Auch ist es schwer, die beiden Teilflächen so genau übereinstimmend mit denen der Prothese zu formen, wie dies bei zementfreier Verankerung der Prothese erforderlich ist.

Der Erfindung liegt daher die Aufgabe zugrunde, eine solche Form der Unterfläche der Auflageplatte zu schaffen, die trotz ihrer Zusammensetzung aus unterschiedlich geneigten Teilflächen in einfacher Weise und mit einfachen Mitteln eine genau übereinstimmende Bearbeitung der Knochenresektionsfläche gestattet, so daß zur insbesondere zementfreien Verankerung der Prothese Identität der zusammenwirkenden Flächen der Prothese und des Knochens gewährleistet ist.

Die erfindungsgemäße Lösung besteht darin, daß die Unterfläche der Auflageplatte eine von der ebenen Gestalt abweichende Rotationsfläche ist.

Dies beruht auf der Erkenntnis, daß die Herstellung der Resektionsfläche als Rotationsfläche mit geeigneten Fräswerkzeugen bei proximal gelegener Rotationsachse sich besonders einfach gestaltet.

Vorteilhafterweise ist die Unterfläche der Auflageplatte eine Rotationsfläche mit quer zur Knochenlängsrichtung von anterior nach posterior verlaufender Drehachse, insbesondere eine Zylinderfläche. Auch eine sphärische Form kann im Hinblick auf die Bearbeitbarkeit und einen sicheren, eindeutigen Sitz der Prothese vorteilhaft sein. Einfache Formen werden bevorzugt.

Nach einem besonderen Merkmal einer Ausführungsart der Erfindung steht die Drehachse normal zu einer Tangentialebene an der Unterfläche der Auflageplatte, wobei diese insbesondere die Gestalt einer Kugelkalotte oder einer Mehrzahl ringförmiger Erhöhungen und Vertiefungen aufweist. Bei einer solchen Lage der Rotationsfläche beziehungsweise ihrer Drehachse läßt sich nämlich eine besonders genaue Fräsung der Resektionsfläche durch Verwendung eines chirurgischen Instruments erreichen, das aus einem rotierbaren Fräser mit einer der Unterfläche der Auflageplatte der Prothese entsprechenden Schneidenform und einem Fräserhalter mit einer zum Fräser passenden Lagereinrichtung und einem diese haltenden, dem Schaft der Endoprothese formentsprechenden Schaft besteht. Ein solcher Fräser ist an sich bekannt (FR-A-1 083 013).

Dabei kann vorgesehen sein, daß die Lagereinrichtung des Fräsers eine gegenüber der Richtung des Prothesenschafts feststehende oder feststellbare Drehachsrichtung hat. Die Übereinstimmung zwischen den Schäften der Prothese und des Fräserhalters möglichst groß sein, damit hinreichende Lagesicherheit für die Fräserachse besteht. Der leichteren Handhabung wegen kann der Fräserhalterschaft im allgemeinen etwas dünner als der Prothesenschaft ausgeführt sein. Auch seine Länge kann etwas geringer sein. In der Ausgestaltung der Einzelheiten kann der Fräserhalterschaft ebenfalls Abweichungen aufweisen, soweit diese die Übereinstimmung der Lage in der Markhöhle des Knochens nicht beeinträchtigen.

Das Merkmal, daß die Unterfläche der Auflageplatte eine Rotationsfläche ist, braucht nicht zu besagen, daß ihre äußere Begrenzung ebenfalls eine Rotationsfläche darstellen muß. Vielmehr kann die Unterfläche nach unterschiedlichen Seiten hin unterschiedlich weit ausladen. Beispielsweise kann vorgesehen sein, daß die Auflagefläche weiter nach medial vorragt als nach lateral. Es ist auch nicht erforderlich, daß die Rotationsachse der Auflagefläche zusammenfällt mit der Achse des Prothesenschafts oder des Prothesenhalses. Wenn die Gestalt der Unterfläche der Auflageplatte winkelige Übergänge von einer Teilfläche zur anderen aufweist, werden diese vorzugsweise im medialen oder mittleren Bereich der Resektionsfläche vorgesehen, um schädliche Kerbwirkungen im Kraftübergangsbereich zum größeren Trochanter zu vermeiden.

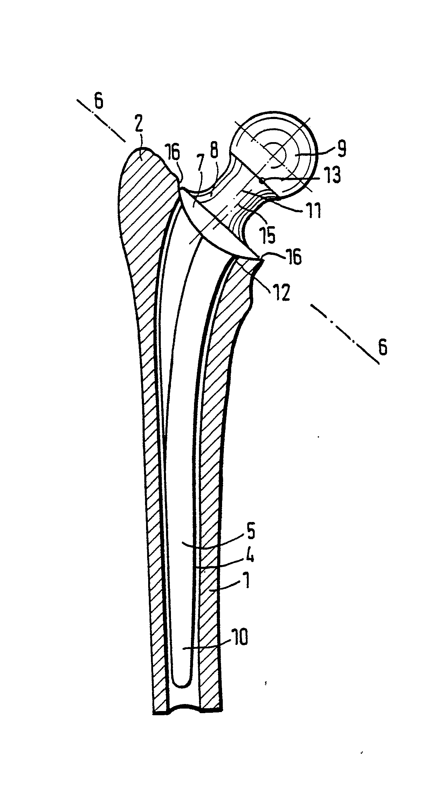

Die Erfindung wird im folgenden näher unter Bezugnahme auf die Zeichnung erläutert, die ein vorteilhaftes Ausführungsbeispiel veranschaulicht. Darin zeigt die einzige Figur einen Oberschenkelknochen mit eingesetzter Prothese.

In der Zeichnung erkennt man schraffiert die Schnittfläche des Knochens 1 mit großem Trochanter 2 und kleinem Trochanter 3 und Markhöhle 4, die zur Aufnahme des Prothesenschafts 5 vorbearbeitet ist. Der Oberschenkelhals ist längs der Ebene reseziert, deren Hauptrichtung durch die strichpunktierte Linie 6 angedeutet ist und die lateral so hoch liegt, daß der große Trochanter 2 erhalten bleibt, während sie medial so tief liegt, daß der Halsteil etwa die dargestellte Länge haben kann, die erforderlich ist, um dem Oberschenkelknochen hinreichende Schwenkbeweglichkeit gegenüber dem Acetabulum zu verleihen. Die Resektionslinie 6 verläuft daher etwa unter einem Winkel von 45° zur Längsrichtung des Oberschenkelknochens 1.

Die Prothese besteht aus dem Schaft 5, der Auflageplatte 7, dem Halsteil 8 und dem Gelenkkopf 9. Der Schaft ist mit oder ohne Knochenzement in die Markhöhle 4 eingesetzt und gibt in seinem distalen Abschnitt 10 die Richtung des Oberschenkelknochens an. Die Achse 11 des Halsteils 8 verläuft unter 45° zur Richtung des Oberschenkelknochens.

Die Unterfläche der Auflageplatte 7 wird von einer Zylinderfläche 12 gebildet, deren Mittelachse bei 13 die Zeichenebene lotrecht durchstößt. In ihrem mittleren Bereich wird sie unterbrochen durch den Ansatz des Schafts 5, während sie außen durch den Rand der Auflageplatte 7 begrenzt ist, die man sich als Kreiszylinderfläche um die Achse 15 vorzustellen hat. Statt der kreisförmigen Randbegrenzung dieser Auflageplatte 4 ist selbstverständlich auch eine rechteckige oder ovale Begrenzung oder andere Gestaltung möglich. Die Auflageplatte 7 mit ihrer zylindrischen Unterfläche 12 ist gegenüber der Mittelachse 11 des Halsteils 8 ein wenig nach medial verschoben, um den medialen Bereich der Unterfläche, der für die Kraftübertragung auf den Knochen wichtiger als der laterale Teil derselben ist, zu betonen.

Die Resektionsfläche 16 des Knochens, die durch die Linie 6 nur in ihrem ungefähren Verlauf bezeichnet ist, ist identisch mit der Unterfläche 12 der Auflageplatte zylindrisch geformt. Der Radius des Zylinders ist so gewählt, daß die Prothesenunterfläche 12 bzw. die Resektionsfläche 16 medial nahezu rechtwinklig zur Oberschenkelknochen-Längsrichtung auslaufen. Man erhält dadurch im Bereich der harten medialen Corticalis eine ausgezeichnete Übertragung der vertikalen Kräfte von der Prothese auf den Oberschenkelknochen. Das gilt (wenngleich auch in geringerem Maße) auch für die weiteren medial von der Mittellinie 15 gelegenen Teile der Unterfläche 12, da sie im Vergleich mit der Linie 6 flacher verlaufen. Hingegen verläuft die Resektionsfläche 16 lateral von der Mittellinie 15 steiler. Dies ist jedoch für die Kraftübertragung von geringerem Belang.

Anhand der Zeichnung kann man sich die Unterfläche 12 der Auflageplatte 7 auch sphärisch mit Krümmungsmittelpunkt 13 gestaltet vorstellen. Die Vorteile einer solchen Ausführung stimmen mit denen einer zylindrischen Ausführung im wesentlichen überein, wobei noch eine Stabilisierung der Prothese in Richtung lotrecht zur Zeichenebene hinzukommt. In beiden Fällen bleibt der Bereich des großen Trochanter 2 unbeeinträchtigt. Es werden auch festigkeitsmäßig nachteilige Kerben vermieden.

Bei zylindrischer Ausführung der Unterfläche 12 und der Resektionsfläche 16 setzen sich diese Flächen aus geradlinigen Erzeugenden zusammen, die lotrecht zur Zeichenebene verlaufen und daher durch eine Fräsung mittels eines lotrecht zur Zeichenebene gerade ausgebildeten oder in dieser Richtung bewegten Werkzeugs mit hoher Genauigkeit hergestellt werden können. Statt dessen ist auch eine Fräsung mittels eines rotierenden Werkzeugs möglich, dessen Rotationsachse lotrecht zur Zeichenebene durch den Krümmungsmittelpunkt 13 verläuft.

In jedem Falle kann der Fräser an einem Halter angeordnet sein, der einen in die vorgearbeitete Markhöhle des Knochens formgenau passend einsetzbaren Schaft aufweist, wodurch Gewähr dafür gegeben wird, daß die von dem Fräser hergestellte Form der Resektionsfläche genau zu der Form der Prothese paßt.

Die gemäss Artikel 76 und Regel 25 EPUe eingereichte Europäische Patentanmeldung Nr. 85 103 542.8, am 04.12.1985 veröffentlicht mit Nummer EP-A1-0 163 042, betrifft hier ausgeschiedene Ansprüche.

**Patentansprüche**

1. Gelenkendoprothese mit einem in die Höhle (4) eines langen Knochens (1) einzusetzenden Schaft (5) und einer den Schaft an dessen dem Gelenk benachbarten Ende abschließenden Auflageplatte (7), deren Unterfläche (12) zur Auflage auf der formentsprechend vorbearbeiteten Resektionsfläche (16) des Knochens (1) bestimmt ist und in ihrem medialen Teil einen durchschnittlich größeren Winkel mit der Richtung des langen Knochens einschließt als in ihrem lateralen Teil, dadurch gekennzeichnet, daß die Unterfläche (12) der Auflageplatte (7) eine von der ebenen Gestalt abweichende Rotationsfläche ist.

2. Gelenkendoprothese, insbesondere femorale Hüftgelenkendoprothese, nach Anspruch 1, dadurch gekennzeichnet, daß die Unterfläche (12) der Auflageplatte (7) eine Rotationsfläche mit quer zur Knochenlängsrichtung von anterior nach posterior verlaufender Drehachse besitzt.

3. Gelenkendoprothese nach Anspruch 2, dadurch gekennzeichnet, daß die Unterfläche (12) der Auflageplatte (7) etwa zylindrisch ist.

4. Gelenkendoprothese nach Anspruch 1, dadurch gekennzeichnet, daß die Drehachse (13) normal zu einer Tangentialebene an der Unterfläche (12) der Auflageplatte (7) steht.

5. Gelenkendoprothese nach Anspruch 4, dadurch gekennzeichnet, daß die Unterfläche eine Kugelkalotte ist.

6. Gelenkendoprothese nach Anspruch 4, dadurch gekennzeichnet, daß die Unterfläche eine Mehrzahl ringförmiger Erhöhungen und Vertiefungen aufweist.

## Claims

1. A joint endoprosthesis with a shaft (5) to be inserted in a cavity (4) of a long bone (1) and with a bearing plate (7) which terminates the shaft at its end nearest the joint and the underside (12) of which is designed to bear against the resection surface (16) of the bone (1), previously prepared to match the shape thereof, and in its medial portion forms with the direction of the long bone an angle which is on average larger than in its lateral portion, characterised in that the underside (12) of the bearing plate (7) is a surface of revolution deviating from a flat shape.

2. A joint endoprosthesis, in particular a femoral hip joint endoprosthesis, according to Claim 1, characterised in that the underside (12) of the bearing plate (7) has a surface of revolution with an axis of rotation extending from anterior to posterior transversely to the longitudinal direction of the bone.

3. A joint endoprosthesis according to Claim 2, characterised in that the underside (12) of the bearing plate (7) is approximately cylindrical.

4. A joint endoprosthesis according to Claim 1, characterised in that the axis of rotation (13) lies normal to a tangential plane on the underside (12) of the bearing plate.

5. A joint endoprosthesis according to Claim 4, characterised in that the underside is a spherical cap.

6. A joint endoprosthesis according to Claim 4, characterised in that the underside has a plurality of annular projections and recesses.

## Revendications

1. Endoprothèse d'articulation comportant une tige (5) destinée à être insérée dans la cavité médullaire (4) d'un os long (1) et une plaque d'appui (7) qui termine la tige à l'extrémité de celle-ci voisine de l'articulation, plaque dont la face inférieure (12) est destinée à prendre appui sur la surface de résection (16) de l'os (1) préparée de manière à correspondre à sa structure et forme, dans sa partie centrale, un angle plus grand en moyenne avec la direction de l'os long que dans sa partie latérale, caractérisée en ce que la surface inférieure (12) de la plaque d'appui (7) est une surface de rotation qui diffère de la forme plane.

2. Endoprothèse d'articulation, en particulier endoprothèse fémorale de la hanche, selon la revendication 1, caractérisée en ce que la surface inférieure (12) de la plaque d'appui (7) est une surface de révolution dont l'axe de révolution s'étend d'avant en arrière perpendiculairement à la direction longitudinale de l'os.

3. Endoprothèse d'articulation selon la revendication 2, caractérisée en ce que la surface inférieure (12) de la plaque d'appui (7) est à peu près cylindrique.

4. Endoprothèse d'articulation selon la revendication 1, caractérisée en ce que l'axe de rotation (13) est normal à un plan tangentiel à la surface inférieure (12) de la plaque d'appui (7).

5. Endoprothèse d'articulation selon la revendication 4, caractérisée en ce que la surface inférieure est une calotte sphérique.

6. Endoprothèse d'articulation selon la revendication 4, caractérisée en ce que la surface inférieure présente en majeure partie des surélévations et des dépressions annulaires.